Europäisches Patentamt

(19) European Patent Office

Office européen des brevets

(11) Publication number: **0 165 300 B1**

(12) **EUROPEAN PATENT SPECIFICATION**

(45) Date of publication of patent specification: **09.11.88**

(21) Application number: **85900374.1**

(22) Date of filing: **06.12.84**

(86) International application number:
**PCT/US84/01991**

(87) International publication number:
**WO 85/02615 20.06.85 Gazette 85/14**

(51) Int. Cl.⁴: **C 07 D 307/89, C 07 C 76/06**

(54) **NITRATION OF PHTHALIC ACID AND PHTHALIC ANHYDRIDE USING NITRIC ACID.**

(30) Priority: **08.12.83 US 559575**

(43) Date of publication of application:
**27.12.85 Bulletin 85/52**

(45) Publication of the grant of the patent:
**09.11.88 Bulletin 88/45**

(84) Designated Contracting States:
**DE FR GB NL SE**

(56) References cited:
**EP-A-0 108 604**
**CH-A- 329 367**
**FR-A-2 270 247**
**GB-A- 635 635**

**Houben-Weyl, Methoden der organischen
Chemie, vol. X/1, part 1, 1971, pp. 622-623**

(73) Proprietor: **GENERAL ELECTRIC COMPANY
1 River Road
Schenectady New York 12305 (US)**

(72) Inventor: **ODLE, Roy, Ray
2386 Burgoyne
Schuylerville, NY 12871 (US)**

(74) Representative: **Schüler, Horst, Dr.
Kaiserstrasse 69
D-6000 Frankfurt/Main 1 (DE)**

Courier Press, Leamington Spa, England.

**Description**

The present invention is concerned with an improved process for making the 3-nitro- and 4-nitro derivatives of phthalic acid without the use of sulfuric acid, involving

(1) mixing phthalic acid or phthalic acid anhydride with a nitric acid having a concentration of at least 95 weight %, and

(2) reacting the mixture at a temperature in the range from 20°C to the temperature at which the said nitric acid boils at the pressure employed to produce 3-nitrophthalic acid and 4-nitrophthalic acid.

Preferably the improved process of the present invention comprises mixing the phthalic acid or the phthalic acid anhydride with a nitric acid having a concentration of at least 97% by weight, more preferably 99% by weight. The temperature range at which the nitration reaction is allowed to proceed is preferably from 50°C to the boiling point of nitric acid, most preferably from 60°C to 85°C, allowing the nitration reaction to proceed to form the nitrated derivatives of the reactant and thereafter recovering such nitrated products by conventional recovering methods known in the art.

Such alternative methods for preparing nitrated derivatives of aromatic compounds are continually being sought as many of these nitrated aromatic compounds are especially useful as starting reactants or intermediates in the preparation of commercial resinous compositions.

Cook et al (U.S. 3,981,933) disclose a process for the preparation of nitrated aromatic compounds having 6 to 18 carbon atoms, which process comprises contacting the aromatic compound, in the presence of methylene chloride as reaction medium, with from 80 to 100% by weight concentrated sulphuric acid and 90 to 100% by weight concentrated nitric acid, advantageously within a temperature range of from −20°C to 50°C, or even somewhat higher, and thereafter isolating the nitro compound.

Bacha et al (U.S. 4,137,419) disclose a process for the preparation of 4-nitro-o-phthalic acid which involves nitrating an indene, polyindene, dihydronaphthaline or polydihydronaphthaline with concentrated nitric acid and thereafter oxidizing said nitrated organic compound with dilute nitric acid, said nitration being conducted with an aqueous nitric acid solution having a concentration of from 70 to 95 weight % at a temperature of −40°C to 90°C and said oxidation being conducted with an aqueous nitric acid solution having a concentration from 5 to 50 weight % at a temperature of 135°C to 210°C. Houben-Weyl, Vol. X/1 pages 622 and 623 discloses a process for preparing 5-nitrophthalic acid by refluxing isophthalic acid with nitric acid. Finally, Cook et al (U.S. 3,887,588) disclose a method for preparing nitrophthalic anhydrides by treating phthalic anhydride in concentrated sulphuric acid with concentrated nitric acid and thereafter extracting the formed nitrophthalic anhydrides with methylene chloride. .

**Summary**

Unexpectedly, it has now been discovered that phthalic acid and phthalic anhydride may be nitrated to form a mixture of 3- and 4-nitrophthalic acid in a nitration process which employs only nitric acid. Specifically, the process of the present invention comprises

(1) mixing phthalic acid or phthalic acid anhydride with a nitric acid having a concentration of at least 95 weight percent, and

(2) reacting the mixture at a temperature in the range from 20°C to the temperature at which the said nitric acid boils at the pressure employed to produce 3-nitrophthalic acid and 4-nitrophthalic acid, and

(3) thereafter recovering the nitrated products by known methods to obtain a mixture composed essentially of the 3- and 4-isomers of nitrophthalic acid.

The weight ratio of the starting reactants, nitric acid to phthalic acid or phthalic anhydride may vary widely. Generally said ratio is from 0.4 to 50, preferably from 5 to 30, most preferably from 9 to 15.

The process of the present invention eliminates the additional requirement and expense of sulfuric acid, is effective over a wide range of temperatures and provides excellent yields. Furthermore, this new process is considered safer and less costly than prior processes, particularly in the event of a cooling failure and/or a runaway reaction. Finally, recovery of the reaction products of the present invention may be by any known method for recovery of nitrated products including drying, evaporation, extraction and the like and is easier since removal of sulfuric acid is no longer a consideration. Rather, the products of the present invention are recovered directly from the nitric acid.

**Detailed description of the invention**

For the purpose of this specification and the appended claims, the "boiling point of nitric acid" is defined as the temperature at which the specific nitric acid used, under the pressure employed, boils. This definition is necessitated by the fact that nitric acids of less than 100% concentration have a higher boiling point than 100% concentrated nitric acid and that the boiling point of nitric acid may be elevated by raising the pressure under which the reaction takes place above atmosphere. Such instances are clearly intended to be within the full scope of the present invention as set forth in this specification and claimed by the appended claims.

The nitric acid useful for the nitration process disclosed herein should have a concentration of at least 95% by weight and is preferably within the range of from 97 to 100% concentration by weight. Nitric acids of lower concentration than 95% are useful for the all nitric acid nitration process; however, the use of such concentrations results in processes which are too slow to be cost effective and therefore do not belong to the invention. Nitric acids of such concentrations are available commercially or may be prepared by known concentrating methods from more widely available commercial nitric acid of 60 to 67% concentration.

The amount of concentrated nitric acid used should be at least of the stoichiometric amount necessary to attach one nitro ($NO_2$) group on the aromatic nucleus of the phthalic acid or phthalic anhydride. Generally, the weight ratio of nitric acid to the phthalic acid or phthalic anhydride should be from 0.4 to 50, preferably from 5 to 30, most preferably from 9 to 15. Obviously, lower or higher amounts of nitric acid may be used in the process of the present invention, however, lower amounts of nitric acid result in poor yields and too slow a reaction rate as to be cost effective, whereas higher amounts of nitric acid may result in unnecessary spoiling of concentrated nitric acid and increased cost for such acid and its recycling.

Phthalic acid and phthalic anhydride useful in the present invention are well known and widely available commercially. Sources for these materials include Mallinckrodt, Inc. of St. Louis, Missouri; Monsanto Company, of St. Louis, Missouri; and Exxon Chemical of Houston, Texas.

The process of the present invention comprises mixing together the concentrated nitric acid and the phthalic acid or phthalic anhydride in a reactor or reactors equipped with a stirrer or agitating means and means for heating or cooling the reactor(s). The reactor(s) may be such as to allow for either batch or continuous processing.

Specific variations in the design of the process systems employable to practice the present invention are known to those skilled in the art. For example, it is possible to use one or more reactors in series or in parallel which operate in the plug flow mode with or without radial mixing and with or without heating or cooling. Alternatively, it is possible to use one or more reactors in series or in parallel which operate in the back mixing mode, again with or without heating and cooling and operating in a batch or continuous mode. Finally, it is also possible to use a combination of reactors with features of both the foregoing.

The mode of mixing and sequence of addition of reactants is not critical to the present invention. Feed of the reactants may either be into the first reactor or be portioned among the reactors if more than one reactor is used, or they may be entered at different locations of the reactor or reactors. Further, the reactants may be pre-mixed before entering the reaction process or they may be fed separately. It is also possible that one or both reactants are brought to the desired reaction temperature prior to mixing or entering the reactor.

The pressure range under which this process operates may vary from vacuum to above atmospheric pressure. Depending on the type of reactor or reactors employed, they may preferentially operate under slight vacuum for process and safety reasons. Otherwise, the process is generally run at about atmospheric pressure.

The reaction temperature should fall within the range of from 20°C. to the boiling point of nitric acid, preferably from about 50°C. to the boiling point of nitric acid, most preferably from about 60°C. to about 85°C. The actual temperature to be employed is dependent upon the desired rate of reaction and the economics of the nitration. More specifically, the higher the temperature the faster the nitration reaction. However, very high temperatures, around the boiling point of nitric acid, should be avoided to prevent the loss of nitric acid due to both boiling and conversion to nitrous oxides.

It should also be noted that temperatures outside the range of temperatures disclosed above could be substituted for those of the process of the present invention. However, lower temperatures result in a reaction rate which is too slow to be cost effective, whereas higher temperatures require operation at above atmospheric pressure to prevent boiling and subsequent loss of nitric acid.

While the temperature at which the reaction is run has the most significant impact on reaction rate, the specific reactants used and the ratio of reactants in the reaction mix also influence the reaction rate and isomer ratio. With respect to the former, the higher the concentration of the nitric acid in the initial mix or as added during continuous processing the faster the reaction rate. Finally, with respect to the ratio of the reactant mix, it is found that the rate of reaction increases as the weight ratio of nitric acid to phthalic acid or phthalic anhydride increases. Also, as the weight ratio increases, the ratio of 4-isomer to 3-isomer in the reaction products decreases, i.e. approaches 1:1.

Thus by varying any one or all of the foregoing, one may significantly increase or decrease the time for which the reaction should run to obtain optimum yield. In general, with a reaction run at a temperature within the preferred range, e.g. 60—85°C., up to about 90% or greater yields may be obtained within three hours. Optionally, these yields may be increased further by allowing the reaction mix to stand for a period of time prior to separation.

As noted above, the pressure under which the reaction is run should generally be about atmospheric pressure. It is possible, though, to vary the reactor pressures. This would be expected to have only minor influence on the reaction rate at the same temperature. However, an increase of both pressure and temperature would be expected to enhance the reaction rate measurably. Thus, as noted above, since the boiling point of nitric acid may be elevated by increasing the pressure in the reaction vessel, such elevated boiling points are intended to be within the full scope of the term "about the boiling point of nitric acid" in the specification and appended claims.

The desired reaction products of the process of the present invention are comprised primarily of the 3- and 4-isomers of nitrophthalic acid. Generally, said ratio varies from about a 1:1 mix to about a 3:2 mix of the 4-isomer to 3-isomer.

The reaction products themselves may be recovered from the reaction mix by any of the known

3

methods for recovery of nitrated products. Exemplary of the methods available include: extraction; evaporation; drying; precipitation and drying and the like. Recovered unreacted phthalic acid and/or phthalic anhydride may be reused and the spoiled or used nitric acid may be recycled by known methods for reuse.

In order that those skilled in the art may better understand how the present invention may be practiced, the following examples are given by way of illustration and not by way of limitation.

Description of the preferred embodiments

Generally, the reaction products were analyzed by High Pressure Liquid Chromatography (HPLC) wherein 50 µl aliquots were quenched into 3.8 mls of a solution comprising 0.5 M tetraethylammonium bromide and 0.96 M sodium acetate. The samples were analyzed at 280 nm on a Waters u Bondpak/$C_{18}$ column using 1.5 ml/min of mobile phase which consisted of a solution of 0.005 M tetraethylammonium bromide, 0.035 M acetic acid and 0.070 M sodium acetate.

Examples 1 and 2

100 parts by weight and 150 parts by weight 99% by weight concentrated nitric acid are each added to a reaction vessel and brought to a temperature of 70°C. To each solution is added 10 parts by weight phthalic acid. Nitration is allowed to continue at 70°C. for three hours and produces yields of greater than 90% of theoretical yield consisting essentially of a mixture of 3- and 4-nitrophthalic acid. Table 1 shows the make-up of the reaction product mix in mole percent. The mole ratio of 4- to 3-nitrophthalic acid formed was approximately 1.1:1.

TABLE 1

|  | Wt. HNO$_3$/ wt. phthalic acid | Reaction product mix (mole %) 3- and 4-NP acid | Phthalic acid |
|---|---|---|---|
| Example 1 | 10/1 | 96.0 | 3.98 |
| Example 2 | 15/1 | 94.4 | 5.62 |

Examples 3—16

A series of experiments are run as in Examples 1 and 2 except that phthalic anhydride is substituted for phthalic acid. The temperature and weight ratio of the reactions are as presented in Table 2. Table 2 also presents the mole % based on the theoretical yield of the products formed during a 3 hour nitration.

4

TABLE 2

| Example | Temperature | Wt. HNO$_3$/ wt. phthalic anhydride | 4- and 3- nitrophthalic acid |
|---|---|---|---|
| 3 | 25 | 5/1 | 12.8(25.6)[a] |
| 4 | 25 | 10/1 | 18.6(38.2)[a] |
| 5 | 25 | 15/1 | 21.6(43.3)[a] |
| 6 | 25 | 20/1 | 23.7(46.0)[a] |
| 7 | 50 | 5/1 | 52.5 |
| 8 | 50 | 10/1 | 68.3 |
| 9 | 50 | 15/1 | 75.2 |
| 10 | 50 | 20/1 | 77.6 |
| 11 | 70 | 10/1 | 93.5 |
| 12 | 70 | 15/1 | 88.0 |
| 13 | 80 | 5/1 | 94.7 |
| 14 | 80 | 10/1 | 99.1 |
| 15 | 80 | 15/1 | 100 |
| 16 | 80 | 20/1 | 100 |

[a] Number in parenthesis represent mole % formed after 7 hours.

Table 2 demonstrates the ability of the all nitric nitration process to be run at various temperatures and weight ratio of starting reactants. It is clear that the higher temperatures are preferred. It is further evident that higher mole ratios of nitric acid to phthalic anhydride favors a faster reaction rate.

Examples 17—19

100 parts by weight of 99.97 and 95% concentrated nitric acid were each added to a reaction vessel and brought to 70°C. To each of these is added 10 parts by weight phthalic anhydride. The nitration reaction is allowed to proceed for 3 hours after which the reaction products comprising the 3- and 4-isomers of nitrophthalic acid and unreacted phthalic acid, as shown in Table 3, are recovered.

TABLE 3

| Example | HNO$_3$ | 3- and 4- nitrophthalic acid | Phthalic acid |
|---|---|---|---|
| 17 | 99 | 93.1 | 6.9 |
| 18 | 97 | 83.5 | 16.5 |
| 19 | 95 | 70.2 | 29.8 |

From Table 3, it is clear that the concentration of the nitric acid greatly influences the reaction rate.

Obviously, other modifications and variations of the present invention are possible in light of the above teachings. It is, therefore, to be understood that changes may be made in the particular embodiments of the invention described which are within the full intended scope of the invention as defined by the appended claims.

## Claims

1. A process for making the 3-nitro- and 4-nitro-derivatives of phthalic acid without the use of sulphuric acid, consisting of

(1) mixing phthalic acid or phthalic acid anhydride with a nitric acid having a concentration of at least 95 weight percent, and

(2) reacting the mixture at a temperature in the range from 20°C to the temperature at which the said nitric acid boils at the pressure employed to produce 3-nitrophthalic acid and 4-nitrophthalic acid, and

(3) thereafter recovering the nitrated products by known methods to obtain a mixture composed essentially of the 3- and 4-isomers of nitrophthalic acid.

2. The process of claim 1 wherein the temperature at which the nitration reaction is allowed to proceed is from 50°C to the boiling point of nitric acid.

3. The process of claim 1 wherein the temperature at which the nitration reaction is allowed to proceed is from 60°C to 85°C.

4. The process of claim 1 wherein the weight ratio of nitric acid to substrate is from 0.4 to 50.

5. The process of claim 1 wherein the weight ratio of nitric acid to substrate is from 5 to 20.

6. The process of claim 1 wherein the weight ratio of nitric acid to substrate is from 9 to 15.

7. The process of claim 1 wherein the nitric acid is at least 97% by weight concentration.

8. The process of claim 1 wherein the nitric acid is at least 99% by weight concentration.

## Patentansprüche

1. Verfahren zur Herstellung der 3-Nitro- und 4-Nitro-derivate von Phthalsäure ohne Verwendung von Schwefelsäure, bestehend aus

(1) Mischen von Phthalsäure oder Phthalsäureanhydrid mit einer Salpetersäure mit einer Konzentration von wenigstens 95 Gew.-% und

(2) Umsetzung der Mischung bei einer Temperatur im Bereich von 20°C bis zur Temperatur, bei der die Salpetersäure unter dem verwendeten Druck siedet, um 3-Nitrophthalsäure und -Nitrophthalsäure zu erzeugen, und

(3) daran anschließend Gewinnung der nitrierten Produkte nach bekannten Verfahren, um eine Mischung zu erhalten, die im wesentlichen aus den 3- und 4-Isomeren der Nitrophthalsäure zusammengesetzt ist.

2. Verfahren nach Anspruch 1, worin die Temperatur, bei der die Nitrierungsreaktion durchgeführt wird, von 50°C bis zum Siedepunkt der Salpetersäure beträgt.

3. Verfahren nach Anspruch 1, worin die Temperatur, bei der die Nitrierungsreaktion durchgeführt wird, von 60°C bis 85°C beträgt.

4. Verfahren nach Anspruch 1, worin das Gewichtsverhältnis von Salpetersäure zum Substrat von 0,4 bis 50 beträgt.

5. Verfahren nach Anspruch 1, worin das Gewichtsverhältnis von Salpetersäure zum Substrat von 5 bis 20 beträgt.

6. Verfahren nach Anspruch 1, worin das Gewichtsverhältnis von Salpetersäure zum Substrat von 9 bis 15 beträgt.

7. Verfahren nach Anspruch 1, worin die Salpetersäure eine Konzentration von wenigstens 96 Gew.-% aufweist.

8. Verfahren nach Anspruch 1, worin die Salpetersäure eine Konzentration von wenigstens 99 Gew.-% aufweist.

## Revendications

1. Procédé de préparation des dérivés nitro-3 et nitro-4 de l'acide phtalique sans employer d'acide sulfurique, constitué par

(1) le mélange d'acide phtalique ou d'anhydride d'acide phtalique avec un acide nitrique présentant une concentration d'au moins 95% en poids et,

(2) la mise en réaction du mélange à une température comprise entre 20°C et la température à laquelle l'acide nitrique bout à la pression employée pour produire l'acide nitro-3 phtalique et l'acide nitro-4 phtalique et ensuite.

(3) la récupération des produits nitrés par des procédés connus pour obtenir un mélange composé essentiellement des isomères-3 et -4 de l'acide nitrophtalique.

2. Procédé selon la revendication 1 dans lequel la température à laquelle on met en oeuvre la réaction de nitration est comprise entre 50°C et le point d'ébullition de l'acide nitrique.

3. Procédé selon la revendication 1, dans lequel la température à laquelle on met en oeuvre la réaction de nitration est comprise entre 60°C et 85°C.

4. Procédé selon la revendication 1, dans lequel le rapport pondéral de l'acide nitrique au substrat est compris entre 0,4 et 50.

**0 165 300**

5. Procédé selon la revendication 1 dans lequel le rapport pondéral de l'acide nitrique au substrat est compris entre 5 et 20.

6. Procédé selon la revendication 1 dans lequel le rapport pondéral de l'acide nitrique au substrat est compris entre 9 et 15.

7. Procédé selon la revendication 1 dans lequel l'acide nitrique présente une concentration d'au moins 97% en poids.

8. Procédé selon la revendication 1 dans lequel l'acide nitrique présente une concentration d'au moins 99% en poids.